(19) Europäisches Patentamt
European Patent Office
Office européen des brevets

(11) **EP 4 306 121 B1**

(12) **EUROPEAN PATENT SPECIFICATION**

(45) Date of publication and mention
of the grant of the patent:
**11.02.2026 Bulletin 2026/07**

(21) Application number: **22382668.6**

(22) Date of filing: **14.07.2022**

(51) International Patent Classification (IPC):
*A61K 36/185* (2006.01)      *A61P 25/00* (2006.01)
*A61P 25/08* (2006.01)      *A61P 25/28* (2006.01)

(52) Cooperative Patent Classification (CPC):
**A61K 36/3482**

(54) **METHOD FOR OBTAINING CANNABIS EXTRACT AND USES THEREOF**

VERFAHREN ZUR ERHALTEN VON CANNABISEXTRAKT UND VERWENDUNGEN DAVON

PROCÉDÉ D'OBTENTION D'EXTRAIT DE CANNABIS ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA TN**

(43) Date of publication of application:
**17.01.2024 Bulletin 2024/03**

(73) Proprietor: **Oils4Cure, S.L.**
**28047 Madrid (ES)**

(72) Inventor: **MORALES ORO, Joaquín**
**E-28047 Madrid (ES)**

(74) Representative: **ABG Intellectual Property Law, S.L.**
**Avenida de Burgos, 16D**
**Edificio Euromor**
**28036 Madrid (ES)**

(56) References cited:
WO-A1-2016/187679      US-A1- 2021 121 435
US-A1- 2021 238 117

# EP 4 306 121 B1

**Description**

## TECHNICAL FIELD OF THE INVENTION

The present invention belongs to the technical field of a method to obtain cannabinoid-containing plant extracts and their use in the prevention or treatment of epilepsy and neuropathic pain.

## BACKGROUND OF THE INVENTION

Epilepsy is a chronic neurological disorder presenting a wide spectrum of diseases that affects approximately 50 million people worldwide. Due to the unpredictability and/or the possible large number of seizure events, epilepsy can be a vastly debilitating disability and may require continuous care of the patients affected.

Recent advances in the understanding of the disease and of the body's internal 'endocannabinoid' system has led to the suggestion that cannabis-base medicines may have the potential to treat this disorder of hyperexcitability in the central nervous system.

The Cannabis genus of flowering plants mainly comprises the *sativa* and *indica* species. Indigenous to Central and South Asia, cannabis was used for millennia to produce hemp fiber for rope, clothing, bowstrings, and paper; for its seeds and seed oils; as livestock feed; and for medicine, religious ceremonies, and recreation. Hemp is now a worldwide crop used to make cordage, construction material, paper, and textiles, as well as for edible seeds, milk, and oil. US 2021/238117 A1 reports on producing cannabidiol from isolated trichomes of cannabis.

The two major neuroactive components in cannabis are the psychoactive Δ9-tetrahydrocannabinol (Δ9-THC) and the nonpsychoactive cannabidiol (CDB). *Cannabis sativa* usually has higher Δ9-THC:CBD ratios than *Cannabis indica.* Δ9-THC activates the endocannabinoid system, which consists of G-protein-coupled cannabinoid (CB) receptors, synthetic and degradative enzymes, and transporters. In the central nervous system, this system influences synaptic communication and modulates eating, anxiety, learning and memory, and growth and development.

Despite the recent advances, the use of cannabinoids in the treatment of epilepsy is still relatively uncommon and the results obtained are mostly not above the preexisting treatments for epilepsy.

Therefore, alternative cannabis based products which provide with more effective treatment of epilepsy are necessary.

## SUMMARY OF THE INVENTION

The present invention describes a new method for obtaining a cannabis extract with improved properties which augment the effect on the treatment of epilepsy.

Therefore, a first aspect of the present invention relates to a method for obtaining a cannabis extract, from here onwards the method of the invention, comprising:

    a) obtaining trichomes from cannabis flowers wherein step a) comprises:

        (i) collecting and drying the cannabis flowers from *Cannabis* plants;
        (ii) cooling the cannabis flowers to a temperature of between 0°C to 10°C, and;
        (iii) washing the cannabis flowers in water at a temperature between about 0°C to about 5°C as to separate the trichomes form the remaining flower;

    b) drying the trichomes of step a) to a moisture content of less than 10% by weight
    c) macerating the trichomes with an C1 - C3 alcohol at a temperature of at least 30°C for at least 48 h, obtaining a first solution;
    d) incubating and stirring the trichomes obtained in step c) with an C1 - C3 alcohol at a temperature of 90°C to 100°C for at least 40 hours, obtaining a second solution;
    e) eliminating at least part of the alcohol present in the second solution, obtaining a extract,

wherein the extract comprises at least about 10% w/v of cannabidiol (CBD) and a concentration of less than 0.5% w/v of tetrahydrocannabinol, and wherein no purification is carried out on the extract.

Another aspect of the present invention relates to a cannabis extract derived from *Cannabis sativa* L. obtained by the method according to the invention.

Yet another aspect relates to a pharmaceutical composition comprising the cannabis extract according to the invention in a therapeutically effective dose and an adequate excipient and/or adjuvant.

A further aspect of the invention relates to the cannabis extract according to the invention for use as a medicine.

Another aspect of the present invention relates to the cannabis extract according to the invention or the pharmaceutical composition according to the invention for use in the prevention and/or treatment of refractory epilepsy and/or of neuropathic pain and spasticity derived from leukodystrophy.

## BRIEF DESCRIPTION OF THE FIGURES

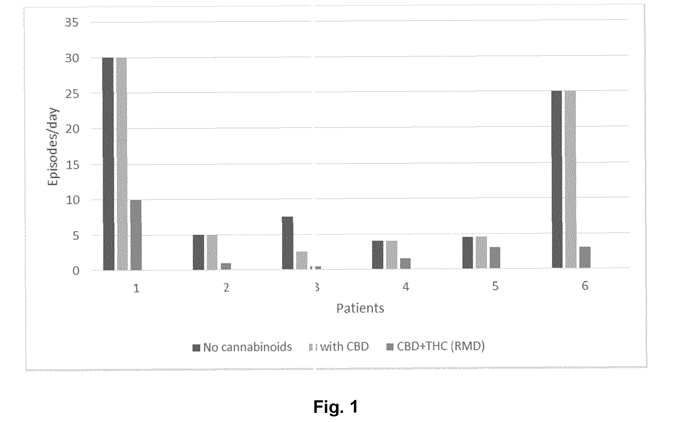

**Figure 1. Observational patient study.** Summary of the results obtained from a study into the effects of the oil RMD (CBD+THC) in the treatment of epilepsy.

## DETAILED DESCRIPTION OF THE INVENTION

The present invention describes a method to obtain a cannabis extract which provides an augmented effect on the treatment of epilepsy, especially refractory epilepsy. The present method is characterized by reducing the change in the terpenes and cannabinoid profile by avoiding high temperatures and pressures during the processing of the cannabis plant or plant parts.

Therefore, a first aspect of the present invention relates to a method for obtaining a cannabis extract, from here onwards the method of the invention, comprising:

a) obtaining trichomes from cannabis flowers flowers wherein step a) comprises:

(i) collecting and drying the cannabis flowers from *Cannabis* plants;
(ii) cooling the cannabis flowers to a temperature of between 0°C to 10°C, and;
(iii) washing the cannabis flowers in water at a temperature between 0°C to 5°C as to separate the trichomes form the remaining flower;

b) drying the trichomes obtained in step a) to a moisture content of less than 10% by weight;
c) macerating the trichomes with an C1 - C3 alcohol at a temperature of at least 30°C for at least 48 h, obtaining a first solution;
d) incubating and stirring the trichomes obtained in step c) with an C1 - C3 alcohol at a temperature of 90°C to 100°C for at least 40 hours, obtaining a second solution;
e) eliminating at least part of the alcohol present in the second solution, obtaining an extract,

wherein the extract comprises at least 10% w/v of cannabidiol (CBD) and a concentration of less than 0.5% w/v of tetrahydrocannabinol, and wherein no purification is carried out on the extract.

The term "cannabis extract" as used herein refers to an extract created by processing raw cannabis plant materials, such as cannabis flowers, to extract and concentrate cannabis compounds, such as cannabinoids and other beneficial components, such as terpenoids and flavonoids. The term "cannabis extract" is used interchangeable with "extract" herein.

The term "Cannabis plant" as used herein encompasses wild type *Cannabis* (including but not limited to the species *Cannabis sativa, Cannabis indica* and *Cannabis ruderalis*) and also variants thereof, including cannabis chemovars (or "strains") that naturally contain different amounts of the individual cannabinoids. For example, some *Cannabis* strains have been bred to produce minimal levels of tetrahydrocannabinol (THC), the principal psychoactive constituent responsible for the high associated with it and other strains have been selectively bred to produce high levels of THC and other psychoactive cannabinoids. *Cannabis* plants produce a unique family of terpeno-phenolic compounds called cannabinoids, some of which produce the "high" one experiences from consuming marijuana.

### Step a) of *the method of the invention*

The method of the invention uses "cannabis flowers" from *Cannabis* plants. In a preferred embodiment of the method of the invention the cannabis flowers used on step a) are from the species selected from *Cannabis sativa, Cannabis indica* and *Cannabis ruderalis,* preferably Cannabis *sativa,* more preferably *Cannabis sativa L.*

The term "cannabis flower" as used herein refers to the part of the plant cannabis composed of the pistils, the sugar leaves and the trichomes. In a preferred embodiment of the method of the invention, the cannabis flowers comprise the pistils, the sugar leaves and/or the trichomes.

The objective of step a) of the method of the invention is to separate the trichomes from the remaining flower constituents.

As used herein, the term "trichomes" refers to crystal-shaped outgrowths or appendages (also called resin glands) on cannabis plants typically covering the leaves and buds. Trichomes produce hundreds of known cannabinoids, terpenes, and flavonoids that make cannabis strains potent, unique, and effective.

*Cannabis* plants can be grouped in varieties. The term "variety" as used herein has identical meaning to the corresponding definition in the International Convention for the Protection of New Varieties of Plants (UPOV treaty), of Dec. 2, 1961, as Revised at Geneva on Nov. 10, 1972, on Oct. 23, 1978, and on Mar. 19, 1991. Thus, "variety" means a plant grouping within a single botanical taxon of the lowest known rank, which grouping, irrespective of whether the conditions for the grant of a breeder's right are fully met, can be i) defined by the expression of the characteristics resulting from a given genotype or combination of genotypes, ii) distinguished from any other plant grouping by the expression of at least one of the said characteristics and iii) considered as a unit with regard to its suitability for being propagated unchanged.

*Cannabis* plants can also be grouped in terms of their contents, or ratios, of chemical compounds. For example, the ratio by weight of CBD to THC in a plant may be used to group *Cannabis* plants into groups. Such groups are called chemotypes, or chemovars. In a more particular embodiment of the method of the invention the cannabis flowers are from a cannabis plant of chemotype III.

The term "chemotype" as used herein refers to a chemically distinct entity in a plant or microorganism, with differences in the composition of the secondary metabolites. Minor genetic and epigenetic changes with little or no effect on morphology or anatomy may produce large changes in the chemical phenotype. In the present disclosure, the term chemotype refers to the content of chemical compounds found in the cannabis plant. This includes, but is not limited to the presence and/or absence of specific cannabinoids found in an extract of the cannabis plant. For example, the cannabidiol (CBD)/ tetrahydrocannabinol (THC) chemotype as used herein refers to the CBD and/or THC content found in the cannabis plant. This also includes the presence or absence of other compounds, including cannabinoids in addition to or other than THC/CBD, and terpenes or terpenoids.

Cannabis plants are organized into 3 main groups or chemotypes organized depending on the ratio of THC to CBD content:

- Type I: THC dominant. This chemotype has a higher concentration of THC than of CBD, having stronger psychoactive effect. Typical ratios of THC:CBD are 100:1, 50:1, 20:1 and 5:1;
- Type II: balanced THC:CBD ratio. This chemotype of cannabis has a roughly equal (1:1) ratio of THC and CBD. Typical ratios of THC:CBD are 4:1, 3:1, 2:1, 1:1, 1:2, 1:3, 1:4;
- Type III: CBD dominant. This chemotype has a higher concentration of CBD than of THC. Typical ratios of CBD:THC are 8:1, 15:1, 30:1 or 25:1.

In a more particular embodiment of the method of the invention, the cannabis flowers are from a cannabis plant of chemotype II, wherein the chemotype II is characterized by a ratio of CBD to THC of 4:1, 3:1, 2:1, 1:1, 1:2, 1:3 or 1:4.

In a more particular embodiment of the method of the invention, the cannabis flowers are from a cannabis plant of chemotype III, wherein the chemotype III is characterized by a ratio of CBD to THC of 30:1 or 25:1.

As used herein, the term "cannabidiol" or "CBD" refers to cannabidiol or cannabidiol prodrugs; pharmaceutically acceptable derivatives of cannabidiol, including pharmaceutically acceptable salts of cannabidiol, cannabidiol prodrugs, and cannabidiol derivatives. CBD includes 2-[3-methyl-6-(I- methylethenyl)-2-cyclohexen-I-yl]-5-pentyl-I,3-benzenediol as well as to pharmaceutically acceptable salts, solvates, metabolites (e.g., cutaneous metabolites), and metabolic precursors thereof. The synthesis of CBD is described, for example, in Petilka et al., Helv. Chim. Acta, 52: 1102 (1969) and in Mechoulam et al., J. Am. Chem. Soc, 87:3273 (1965). In a particular embodiment, the cannabidiol is the compound of formula 2-[3-methyl-6-(I- methylethenyl)-2-cyclohexen-I-yl]-5-pentyl-I,3-benzenediol.

The term "tetrahydrocannabinol" (THC) as used according to the present invention refers to a compound having the chemical name, according to the nomenclature of the International Union of Pure and Applied Chemistry (IUPAC), of (6aR ,10aR )-6,6,9-Trimethyl-3-pentyl-6a,7,8,10a-tetrahydro-6H -benzo[c]chromen-1-ol.

Step a) of the method of the invention comprises a first substep of collecting and drying flowers form *Cannabis* plants. In a more particular embodiment the drying of the *Cannabis* flowers is carried out at a temperature of between about 10°C to about 25°C, preferably between about 15°C to about 18°C, more preferably about 16°C, about 18°C. In another particular embodiment the drying of the *Cannabis* flowers is carried out at a relative humidity of between about 40% to about 70%, preferably between about 50% to about 60%, preferably about 51%, about 52%, about 53%, about 54%, about 55%, about 56%, about 57%, about 58%, about 59%. In yet another particular embodiment the drying of the *Cannabis* flowers is done during at least about 4 days, about 5 days, about 6 days, about 7 days, about 8 days, about 9 days, preferably about 7 days.

In a particular embodiment the drying of the *Cannabis* flowers of the first substep of step a) of the method of the invention is carried out at a temperature of between about 15°C to about 18°C, a relative humidity of about 50% to about 60% and during at least about 5 days, about 6 days, about 7 days, about 8 days.

Once the flowers are collected and dried, step a) of the method of the invention further comprises a second substep of washing the *Cannabis* flowers in water as to separate the trichomes form the remaining flower at a temperature between 0°C to 5°C, preferably at a temperature of 1°C, 2°C, 3°C, 4°C. Besides separating the trichomes form the flowers, this step also allows for removing from the cannabis flowers all material and substances which are foreign and not required for the process of obtaining the extract. Step a) of the method of the invention is performed with water. During the process of washing, the trichomes are separated from the flowers due to the low temperature of the washing process. As to avoid as much as possible detrimental effects on the cannabis plant material during the washing process, the washing process should be done as gently as possible.

Step a) of the method of the invention further comprises a step of cooling the cannabis flowers to a temperature of between 0°C to 10°C which is carried out before the second substep. Several methods are known in the art, as the skilled person is aware, to cooling the cannabis flowers to said temperature. One such method comprises placing cannabis flowers in extraction bags together with ice and placing said bags in a freezer. In a particular embodiment, step a) of the method of the invention further comprises a step previous to the second substep of step a), of cooling the cannabis flowers to a temperature of between about 0°C to about 10°C, wherein the cooling is achieved by placing cannabis flowers and ice in extraction bags, which are placed in a freezer. In a more particular embodiment the extraction bags comprise cannabis flowers and ice in a ratio of 1:2. In yet another particular embodiment the extraction bags comprise about 60 g of cannabis flowers and 120 g of ice.

The term "extraction bags" refers to bags compose of a mesh like material which allows for some of the content of said bag to exude to the exterior. The exudate will depend on the size of the mesh of the material that composes the extraction bag. In the present invention, the exudate will be composed mostly of trichomes which are released from the *Cannabis* flowers. In a particular embodiment the extraction bags have a mesh with openings of less than or equal to 250 micrometers ($\mu$m).

In another particular embodiment of the second substep of step a) of the method of the invention, the wash is done in a reactor by stir cooling the *Cannabis* flowers at a temperature of between about 0°C to about 5°C. In another particular embodiment of the second substep of step a) of the method of the invention, the reactor comprises cannabis flowers and ice cubes in a ratio of 1:20. In another particular embodiment of the second substep of step a) of the method of the invention, the reactor comprises about 1 Kg, about 3 Kg, about 6 kg, about 9 Kg of cannabis flowers and about 20 Kg, about 60 Kg, about 120 Kg, about 180 Kg of ice cubes. The cannabis flowers placed in the reactor may be the *Cannabis* flowers previously placed in extraction bags, which provides the benefit of not having to gather all the cannabis flowers once the wash is complete.

The term "reactor" as used herein refers to a device for containing or controlling a reaction (e.g., a chemical reaction).

The duration of the second substep of step a) of the method of the invention will be dependent on how much or how little foreign or unwanted material is required to be removed from the cannabis flowers. In a particular embodiment of the second substep of step a) of the method of the invention, the washing is carried out during about 30 min to about 6 hours, preferably about 1 hour, about 2 hours, about 3 hours, about 4 hours, about 5 hours.

In a particular embodiment, the second substep of step a) further comprises the step of warming up the mixture to room temperature followed by stopping the stirring. The term "room temperature" as used herein, refers to a range of temperatures, which are considered safe and optimal for working conditions and/or living conditions. According to WHO 2018 guidelines, the room temperatures vary from 18°C to 32°C.

Once washed, the supernatant is discharged while the cannabis flower material is collected, removing as much liquid of the wash as possible. Since during the washing of the cannabis flowers, the trichomes become separated from the flowers, such material may be recovered by filtering the supernatant before discharging it. In a particular embodiment, step a) of the method of the invention further comprises the step of filtering the supernatant after the wash of the second substep of step a) is complete. In a more particular embodiment the filtering is done with a filter with pore sizes of at least about 12 $\mu$m, about 15 $\mu$m, about 18 $\mu$m, about 21 $\mu$m, about 24 $\mu$m, about 27 $\mu$m, about 30 $\mu$m, about 33 $\mu$m, preferably about 23 $\mu$m. In a more particular embodiment of step a) of the method of the invention, the product obtained from the filtering of the supernatant after the wash of the second substep of step a) is complete, is washed with water.

In a particular embodiment, the step a) of the method of the invention further comprises the draining of the trichomes after the washing. In a more particular embodiment the draining is carried out in an inert atmosphere by vacuum filtration, wherein the inert atmosphere is obtain by replacing oxygen with an inert gas selected from nitrogen and argon, preferably argon. In a particular embodiment the vacuum filtration is carried out for at least about 5 min, at least about 10 min, at least about 15 min, at least about 20 min, at least about 25 min.

The term "vacuum filtration", also known as "suction filtration", as used herein refers to a filtration process that uses the flow of a liquid to remove the air contained in the filtering container as to create a negative pressure underneath the filter, which leads to a faster drain of the liquid material present in the filter. The filtering is faster due to the differential of pressure between the inside of the container, which is the recipient of the filtered liquid and the top of the filter where the solid material is collected.

*Step b) of the method of the invention*

Once step a) is complete and the trichomes have been washed, and possibly drained of washing liquid, the next step of the method of the invention comprises drying the cannabis material obtained from step a). Said cannabis material is also called "cake". Step b) of the method of the invention refers to a process of removing moisture content from the cannabis material washed in the previous step. Said process is carried out as to obtain a loss on drying of less than 10 %. In a particular embodiment of the method of the invention, the drying of step is carried out in a clean room, preferably wherein the cake is spread on trays and air dried, at a temperature of between about 18°C to about 40°C about 8 hours, about 12 hours, about 16 hours, about 24 hours, about 32 hours, about 40 hours, about 48 hours, about 56 hours, about 64 hours, about 72 hours, preferably 48 hours. In another particular embodiment of the method of the invention, the drying of step b) is carried out preferably in a clean room, more preferably wherein the cake is spread on trays, by air drying, at a temperature of between about 30°C to about 35°C for about 8 hours, about 12 hours, about 16 hours, about 24 hours, about 32 hours, about 40 hours, about 48 hours, about 56 hours, about 64 hours, about 72 hours, preferably 48 hours. It will be obvious for the skilled in the art, that the required time for drying will be dependent on obtaining a loss on drying of less than 10%, and therefore is should be adjusted either with more or less time, as to accomplish said goal.

The term "loss on drying" as used herein denotes the amount of moisture loss (% by weight) when the target cannabis material, i.e. the trichomes, is dried. It is usually used as an indication of the moisture content of the trichomes, in other words, the dry degree of the trichomes. The loss-on-drying of the trichomes is preferably less than 10 %. The fact that the trichomes have a loss-on-drying of less than 10% denotes that the moisture content of the trichomes is less than 10% by weight when the target trichomes is dried according to the present description of step b), taking the weight of cannabis cake before drying as 100 % by weight. In other words, the moisture content of the target trichomes (the amount of moisture lost when the trichomes is dried) is less than 10% by weight based on 100 % by weight of the target trichomes. The loss-on-drying of the trichomes is preferably less than 10 %, less than 9 %, less than 8 %, less than 7 %, less than 6 %, less than 5 %, less than 4 %, less than 3 %, less than 2 %, less than 1 %, less than 0.5 %.

In a particular embodiment of step b) of the method of the invention, the trichomes are sieved before drying. The term "sieved" as used herein refers to the process of controlling the particle size distribution of the trichomes obtained from step a). The trichomes can then be placed in trays as to perform the drying process.

*Step c) of the method of the invention*

Once the trichomes of the previous method are dried with the correct amount of loss on drying, the next step of the invention concerns the maceration of the dried trichomes obtained in step b) with a C1-C3 alcohol at a temperature of 30°C, during at least 24 hours, at least 48 hours, at least 72 hours, preferably 48h.

The term "macerating" as used herein refers to a process of softening or breaking into pieces a certain material with a liquid. In the present case, said material refers to the trichomes and said liquid refers to alcohol.

Step c) of the method of the invention results in a first solution.

Step c) of the method of the invention is carried out by macerating the plants in a C1-C3 alcohol. In another particular embodiment of step c) of the method of the invention the alcohol is selected from a group consisting of: methanol, ethanol, propanol, isopropanol and any combinations thereof. In an even more particular embodiment, the alcohol is ethanol.

Of importance when selecting the alcohol is its level of purity, as to avoid the introduction of contaminants into the extract. Therefore, in another particular embodiment of step c) of the method of the invention the alcohol has a degree of purity of at least 96% volume/volume (v/v), at least 97% v/v, at least 98% v/v, at least 99% v/v, at least 99,5%, at least 100% v/v, preferably 99.5% v/v.

In a particular embodiment, the alcohol used in step c) of the method of the invention is 99.5% ethanol.

In a particular embodiment, step c) of the method of the invention comprises incubating and stirring the dried trichomes obtained in step b) with 99.5% ethanol at a temperature of about 30°C for 48 h.

The term "purity" as used herein refers to the chemical purity of a substance, i.e., the measurement of the amount of impurities found in a chemical sample. Therefore, an alcohol which has a degree if purity of 96% v/v, means that said solution is composed by at least 96% of pure alcohol and may contain up to 4% of other elements (also called contaminants).

*Step d) of the method of the invention*

Once the first solution is obtained in step b) of the method of the invention, the next step relates to the process of decarboxylation of the cannabis plant material by incubation and stirring of the trichomes in a C1-C3 alcohol at a temperature of between about 90°C to about 100°C, preferably about 91°C, about 92°C, about 93°C, about 94°C, about 95°C, about 96°C, about 97°C, about 98°C, about 99°C, for at least at least about 40 hours, at least about 48 hours, at least about 50 hours, at least about 52 hours, at least about 54 hours, at least about 56 hours, at least about 58 hours, at least about 60 hours, preferably at least about 40 hours. In a particular embodiment, the incubation and stirring of the trichomes in a C1-C3 alcohol is done at a temperature of 90°C to 100°C for at least 40-48 hours.

The term "decarboxylation treatment" as used herein refers to a process step wherein *Cannabis* plant material has been treated such that the cannabinoid acids present in the untreated *Cannabis* plant material have been transformed into the corresponding free cannabinoids. Decarboxylation is usually carried out by heating the *Cannabis* plant material. The term "decarboxylation" literally means removal of the COOH group and its replacement with a proton. Decarboxylation is one of the oldest organic reactions, since it often entails simple pyrolysis, and volatile products distilled from the reactor. Heating is required because the reaction is less favorable at low temperatures. Upon heating, Δ9-tetrahydrocannabionolic acid decarboxylates to give the psychoactive compound Δ9-tetrahydrocannabinol, while cannabidiolic acid decarboxylates to give cannabidiol.

Step d) is carried out in a C1-C3 alcohol. Alcohol has previously been defined for step c) and said definition and related embodiments are equally valid for step d). In a particular embodiment of the method of the invention, the alcohol of step d) is identical to the alcohol of step c).

In a particular embodiment, the incubation and stirring of the trichomes is done in 99.5% ethanol at a temperature of 90°C to 100°C for at least 40-48 hours.

The result of step d) of the method of the invention is a second solution. Said second solution is characterized by comprising a certain content of CBD compared to the sum of the weight of CBD and cannabidiol acid (CBDA). In a particular embodiment of step d) of the method of the invention, the second solution comprises at least about 95 % weight of CBD, at least about 96 % weight of CBD, at least about 97 % weight of CBD, at least about 98 % weight of CBD, at least about 99 % weight of CBD, at least about 100 % weight of CBD compared to the sum of the weight of CBD and CBDA. In another particular embodiment the extract comprises at least about 98 % weight of CBD compared to the sum of the weight of CBD and CBDA.

The expression "compared to the sum of weight CBD and CBDA" as used herein refers to the ratio of the weight of CBD in relation to the sum of the weights of CBD and CBDA expressed in percentages, i.e, CBD/(CBD+CBDA) x 100.

The measurement of CBD, also called the "CBD assay", can be accomplished by High-performance liquid chromatography as exemplified in the Examples further below.

The amount of CBD in relation to CBD+CBDA will depend on several factors such as the exact amount of CBDA in the started cannabis material, the temperature and time of the decarboxylation time. Therefore, once step d) is finished and the CBD ratio to CBD+CBDA determined, it might be desirable to increase the ratio of CBD and therefore, the incubation and stirring of step d) may be continued for a further 2 hours. Therefore, in a particular embodiment of the method of the invention, step d) further comprises at least one, at least two, at least three or more extensions of the incubation and stirring time of at least 2 hours once the original time is finished until the extract comprises at least about 98 % weight of CBD compared to the sum of the weight of CBD and CBDA.

Once the desired degree of CBD % of weight compared to the sum of weight CBD and CBDA has been obtained in step d), the first solution suspension can be further filtered and washed. In a particular embodiment the method of the invention further comprises a step after step d) and before step e) of filtering the first solution obtained in step d). In a particular embodiment the filtering is done by vacuum filtration. In another particular embodiment the filtering is done with filters with a pore size of about 55 μm, about 50 μm, about 45 μm, about 40 μm, about 35 μm, about 30 μm, about 25 μm, about 23 μm, preferably 50 μm.

In yet another particular embodiment, the method of the invention further comprises a step after step d) and before step e) of washing the first solution obtained in step d). In another particular embodiment the washing is done with alcohol, preferably ethanol.

In another particular embodiment, the method of the invention further comprises a step after step d) and before step e) of cooling the first solution obtained in step d) or of the filtrate obtained from the first solution. The term "cooling" as used herein refers to the process of bringing a solution/material to a desired temperature which is below the original temperature at which the solution/material was before the initiation of the process of cooling. The cooling can passive or active, i.e., if the original temperature of the solution/material is above room temperature, then the use of energy to bring down the temperature of the solution/material may not be required, since by the laws of thermodynamic the temperature will of the solution/material will, with time, equalize with the temperature of the room. In another case, if the temperature is below room temperature, then energy is required to bring the temperature down, use methods part of the general knowledge of the skilled person, such as placing the solution/material in a fridge or freezer. In a particular embodiment, the cooling is to room temperature. In another particular embodiment the cooling is to a temperature of about 5°C, of about 6°C, of about 7°C, of about 8°C.

*Step e) of the method of the invention*

Once obtained the first solution of step d), the next step of the method of the invention, step e) has the purpose of partially or completely eliminating the alcohol present in the first solution, obtaining an extract.

Methods to eliminate the alcohol present in the first solution are well known in the art, and the skilled person would be well aware of said methods. In a particular embodiment of the method of the invention, step e) is carried out by evaporation. In a more particular embodiment the evaporation is carried out in a rotary evaporator at a temperature of between about 75°C to about 95°C, preferably about 76°C, about 77°C, about 78°C, about 79°C, about 80°C, about 81°C, about 82°C, about 83°C, about 84°C, about 85°C, about 86°C, about 87°C, about 88°C, about 89°C, about 90°C, about 91°C, about 92°C, about 93°C, about 94°C. In a particular embodiment, evaporation is carried out in a rotary evaporator at a temperature of about 85°C, during at least 2 hours, at least 3 hours, at least 4 hours. As the skilled person will know the time required to carry out the evaporation will be dependent of the desired level of elimination of the alcohol present in the extract. Therefore, the skilled person will be capable of increasing or decreasing the time required for the evaporation.

The term "rotary evaporator" as used herein refers to a device used for the efficient and gentle removal of solvents from samples by evaporation.

During the evaporation an adequate excipient can be added to the extract. Therefore, in a particular embodiment during the evaporation of step e) of the method of the invention, an adequate excipient is added to the extract and the evaporation is continued for at least about 2 hours.

As used herein, the term "excipient" may refer to an additional ingredient that is relatively inert and that is not expected to have biological activity in humans. It will be understood, however, that certain excipients may still have nutritional value. For example, an excipient is selected from the group of diluents, binders, glidants, lubricants, colouring agents and disintegrants. The excipient material itself may be composed of one or more materials selected from the group of sugar alcohols, polyols (e.g. sorbitol, mannitol, xylitol), crystalline sugars, monosaccharides (e.g. glucose, arabinose), disaccharides (e.g. maltose, saccharose, dextrose, lactose), oligosaccharides (e.g. dextrins, cyclodextrins), polysaccharides (e.g. cellulose starch and derivatives thereof), inorganic salts (e.g. sodium chloride, calcium carbonate, magnesium carbonate, talc), and organic salts (e.g. sodium lactate, magnesium stearate).

In another particular embodiment during the evaporation the of step e) of the method of the invention, an adequate excipient is added to the extract and the evaporation is continued for at least about 2 hours, wherein the adequate excipient is medium-chain triglycerides and the amount of the excipient is between about 100 g to about 300 g, preferably about 200 g.

In the context of the present invention, the term "medium-chain triglycerides" or "MCTs" refers to triglycerides with two or three fatty acids having an aliphatic tail of 6-12 carbon atoms, i.e. medium-chain fatty acids (MCFAs). MCTs are found in palm kernel oil and coconut oil and can be separated by fractionation. Examples of MCTs are caproic acid, caprylic acid, capric acid and lauric acid. In a particular embodiment the MCTs are selected from a group consisting of caproic acid, caprylic acid, capric acid, lauric acid and any combination thereof.

In a particular embodiment of the method of the invention the extract comprises less than 1% v/v of a C1-C3 alcohol, preferably less than 1% v/v, less than 0.9% v/v, less than 0.8% v/v, less than 0.7% v/v, less than 0.6% v/v, less than 0.5% v/v, less than 0.4% v/v, less than 0.3% v/v, less than 0.2% v/v, less than 0.1% v/v, less than 0.1% v/v, less than 0.05% v/v of a C1-C3 alcohol. In another particular embodiment of the method of the invention the extract is devoid of a C1-C3 alcohol.

It is desirable that the extract comprises less than 1% v/v a C1-C3 alcohol. Therefore, in a particular embodiment of the method of the invention, step e) can be repeated as many times as needed until the extract comprises less than 1% v/v a C1-C3 alcohol.

In another particular embodiment, the method of the invention further comprises a step of adding to the extract an adequate excipient.

In a particular embodiment, the method of the invention further comprises a step of adding to the extract an adequate excipient, wherein the excipient is medium-chain triglycerides.

In another particular embodiment of the method of the invention, the adequate excipient is added to the extract by a method comprising the following steps:

(i) determining the CBD assay value (A) of the extract;
(ii) weighting the extract (B);
(iii) calculating the amount of excipient to add to the extract using the following

$$\text{formula } excipient \ amount \ (C, grams) = \frac{B \times (A - 20)}{20};$$

(iv) adding excipient to the extract obtained in step (iii) and stirring for between about 5 min to about 10 min;
(v) filtering the extract obtained in (iv) with a Nutsche filter, through a filter with a pore size of 50 $\mu$m, followed by filtering through a filter with a pore size of 19 $\mu$m;
(vi) washing the extract obtained in step (v) with excipient, wherein the amount of excipient to be added is calculated using the following formula

$$excipient\ amount\ (D, grams) = \frac{B \times (A - 17)}{17} - C;$$

(vii) filtering the extract obtained in (vi) with a Nutsche filter, through a filter with a pore size of 5 μm;
(viii) washing the extract obtained in step (vii) with excipient, wherein the amount of excipient to be added is calculated using the following formula

$$excipient\ amount\ (grams) = \frac{B \times (A - 14)}{14} - C - D;$$

(ix) stirring the extract obtained in step (viii) for between about 5 min to about 10 min at room temperature, obtained a new extract.

The extract obtained in step e) (optionally including the excipient) of the method of the invention is characterized by comprising a concentration of at least 10 % w/v of cannabidiol (CBD) and a concentration of less than 0.5% w/v of tetrahydrocannabinol. In a particular embodiment of the method of the invention the extract comprises a concentration of about 10.00 % w/v of CBD, about 10.01 % w/v of CBD, about 10.02 % w/v of CBD, about 10.03 % w/v of CBD, about 10.04 % w/v of CBD, about 10.05 % w/v of CBD, about 10.06 % w/v of CBD, about 10.07 % w/v of CBD, about 10.08 % w/v of CBD, about 10.09 % w/v of CBD, about 10.10 % w/v of CBD, about 10.11 % w/v of CBD, about 10.12 % w/v of CBD, about 10.13 % w/v of CBD, about 10.14 % w/v of CBD, about 10.15 % w/v of CBD, about 10.16 % w/v of CBD, about 10.17 % w/v of CBD, about 10.18 % w/v of CBD, about 10.19 % w/v of CBD, about 10.20 % w/v of CBD, about 10.21 % w/v of CBD, about 10.22 % w/v of CBD, about 10.23 % w/v of CBD, about 10.24 % w/v of CBD, about 10.25 % w/v of CBD, about 10.26 % w/v of CBD, about 10.27 % w/v of CBD, about 10.28 % w/v of CBD, about 10.29 % w/v of CBD, about 10.30 % w/v of CBD, about 10.31 % w/v of CBD, about 10.32 % w/v of CBD, about 10.33 % w/v of CBD, about 10.34 % w/v of CBD, about 10.35 % w/v of CBD. In a particular embodiment, the extract obtained by the method of the invention is characterized by comprising a concentration of CBD comprised between 10.00% w/v and 10.35% w/v of CBD.

In another particular embodiment of the method of the invention, if the extract has a concentration of more than about 10.35 % w/v of CBD, the method of invention further comprises a step of diluting the extract until a concentration of CBD between about 10% to about 10.35% w/v with an adequate excipient, preferably wherein the excipient is the same as added during evaporation, more preferably wherein the excipient is medium-chain triglycerides.

In a particular embodiment of the method of the invention, if the extract has a concentration of more than about 10.35 % w/v of CBD, the method of invention further comprises a step of diluting the extract with an adequate excipient, wherein the excipient is medium-chain triglycerides.

In another particular embodiment of the method of the invention, if the extract has a concentration of more than about 10.35 % w/v of CBD, the method of invention further comprises a step of diluting the extract with an adequate excipient, wherein said step of diluting is carried out by a method comprising the following steps:

(i) determining the CBD assay value (E) of the extract;
(ii) weighting the extract (F);
(iii) calculating the amount of excipient to add to the extract using the following

$$formula\ excipient\ amount\ (grams) = \frac{E \times (F - 10.35)}{10.35};$$

(iv) adding excipient to the extract obtained in step (iii) and stirring for between about 5 min to about 10 min, obtained a new extract.

The extract of the method of the invention is further characterized by the concentration of tetrahydrocannabinol. The extract has a concentration of less than 0.5% w/v of tetrahydrocannabinol. The expression "about 0.5% w/v of tetrahydrocannabinol" as used herein refers to the concentration of tetrahydrocannabinol present in the extract and should be interpreted as including 0.5% w/v (equivalent to 5 mg/mL) or less of tetrahydrocannabinol in the extract.

While in other methods of extracting cannabis plants the extract obtained is submitted to further steps in order to purify the cannabinoids from the remaining compounds, the present method does not require said purification steps. In fact, the inventors have found that the raw extract has a better effect in the treatment of diseases than the purified extract. Therefore, in a particular embodiment of the method of the invention, no purification is carried out on the extract. In yet another particular embodiment of the method of the invention, the CBD and tetrahydrocannabinol components present in the extract are not purified.

As used herein the term "purifying" (and purification) refers to a process of physical separation of a chemical substance of interest from foreign or contaminating substances. Pure results of a successful purification process are termed an "isolate." The following non-exhaustive list of chemical purification methods can be employed within the context of the present invention: filtration, centrifugation, evaporation, liquid-liquid extraction, crystallization, recrystallization, trituration, adsorption, chromatography, and distillation.

The extract can be for immediate use or can be stored in conditions which preserve its chemical properties. In a particular embodiment, the method of the invention further comprises a step of packaging the extract, wherein the packaging is done in containers which protect the extract from light. Examples of containers adequate for storing the extract are bottles of amber glass.

In a further embodiment, the method of the invention further comprises the step of storing the extract, wherein the storage is done at a temperature of less than 5°C under inert atmosphere, wherein the inert atmosphere is obtain by replacing oxygen with an inert gas selected from nitrogen and argon.

The method of the invention is characterized by not using high temperatures or high pressures for obtaining the extract as to avoid the modification of the chemical profile of the extract components.

Therefore, in a particular embodiment the method of the invention is carried out at temperatures of less than about 85 °C and at pressures of less than 0.345 atmospheres.

The term "atmospheres" as used herein refers to the pressure exerted by the weight of the atmosphere, which at sea level has a mean value of 101,325 pascals (roughly 14.6959 pounds per square inch), corresponding to 1 atmosphere.

*Products of the invention*

The method of the invention results in an extract which has different uses. Therefore, another aspect of the present invention relates to a cannabis extract derived from *Cannabis sativa* L. obtained by the method of the invention, from here onwards the extract of the invention.

All previous definitions and embodiments described in relation to the method of the invention are equally valid for the current aspects and their embodiments.

The extract of the invention comprises at least 10.00 % w/v of CBD (100 mg/mL of CBD) and less than 0.5 % w/v (5 mg/mL) of tetrahydrocannabinol. In a particular embodiment, the extract of the invention is characterized by comprising a concentration of CBD comprised between 10.00% w/v and 10.35% w/v and less than 0.5% w/v of tetrahydrocannabinol.

In another particular embodiment of the extract of the invention, the *Cannabis sativa* L. is of chemotype II, wherein the chemotype II is characterized by a ratio of CBD to THC of 4:1, 3:1, 2:1, 1:1, 1:2, 1:3 or 1:4.

In a more particular embodiment of the extract of the invention, the *Cannabis sativa* L. is of chemotype III, wherein the chemotype III is characterized by a ratio of CBD to THC of 30:1 or 25:1.

The extract of the invention can also be used as a pharmaceutical composition. Therefore, another aspect of the present invention relates to a pharmaceutical composition comprising the extract of the invention in a therapeutically effective dose and an adequate excipient and/or adjuvant, from here onwards the pharmaceutical composition of the invention.

The expression "therapeutic effective dose" as used herein refers to an amount of an active agent (i.e., an ingredient such as CDB, THC or the extract of the invention) high enough to deliver the desired benefit, either the treatment or prevention of the illness, but low enough to avoid serious side effects within the scope of medical judgment. The particular dose of the active compound administered according to this invention will of course be determined by the particular circumstances surrounding the case, including the compound administered, the route of administration, the particular condition being treated, and the similar considerations. In a particular embodiment the therapeutic effective dose of the pharmaceutical composition of the invention is of about 0.01 milligrams (mg) per kilo per day to about 3.5 mg per kilo per day of CBD and 0.001 milligrams (mg) per kilo per day to about 0.2 mg per kilo per day of THC, preferably 2.3 mg per kilo per day of CBD and 0.11 mg per kilo per day of THC.

The term "adjuvant" as used herein refers to any substance that enhances the response of a given substance. In the present invention, said term refers to any substance that enhances the effects of the pharmaceutical composition of the invention; it can refer to any adjuvant known by the person skilled in the art.

*Medical uses of the invention*

As previously mentioned the extract and the pharmaceutical composition of the invention find uses related to the treatment of epilepsy.

As such, another aspect of the present invention relates to the extract of the invention for use as a medicine.

Another aspect of the present invention related to the extract of the invention or the pharmaceutical composition of the invention for use in the prevention and/or treatment of refractory epilepsy and/or of neuropathic pain and spasticity derived from leukodystrophy, from here onwards the medical use of the invention.

The term "prevention", as used herein, relates to the capacity to prevent, minimize, or hinder the onset or development of a disease or condition before its onset. When referred to ischemia injury, the term "prevention" also encompasses the administration the protein of the invention prior to the onset of ischemia as well as the administration of the protein of the invention after the onset of ischemia but before the damages caused by ischemia appear.

As used herein, the terms "treat", "treatment", "treatment", or "amelioration". The term refers to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (i.e., not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment).

The term "refractory epilepsy" as used herein refers to drug-resistant epilepsy, i.e., epilepsy which does not respond to treatment. Refractory epilepsy can be cause by several diseases or syndromes or may be of unknown cause.

In a particular embodiment of the medical use of the invention, the extract for use in the prevention and/or treatment of refractory epilepsy is derived from a *Cannabis sativa* L. plant of chemotype III, wherein the chemotype III is characterized by a ratio of CBD to THC of 30:1 or 25:1.

In another particular embodiment of the medical use of the invention, the extract for use in the prevention and/or treatment of neuropathic pain and spasticity derived from leukodystrophy is derived from a *Cannabis sativa* L. plant of chemotype II, wherein the chemotype II is characterized by a ratio of CBD to THC of 4:1, 3:1, 2:1, 1:1, 1:2, 1:3 or 1:4.

In a particular embodiment of the medical use of the invention, the refractory epilepsy is pediatric refractory epilepsy.

The term "pediatric" as used herein refers to children up to 16 years old.

In a particular embodiment of the medical use of the invention, the refractory epilepsy is caused by a disease selected from a group consisting of: refractory epilepsy without known cause, West syndrome, sclerosis tuberosa, MECP2 duplication syndrome, encephalic epilepsies post septic meningitis, Dravet syndrome, Lennox-Gastaut syndrome and Doose syndrome.

The term "West syndrome" as used herein refers to a disease characterized by a constellation of symptoms, namely epileptic/infantile spasms, abnormal brain wave patterns called hypsarrhythmia and intellectual disability. The spasms may range from violent jackknife or "salaam" movements where the whole body bends in half, or they may be no more than a mild twitching of the shoulder or eye changes. These spasms usually begin in the early months after birth and can sometimes be helped with medication. They can also occur in older patients; if this happens, they are called "epileptic spasms" rather than infantile spasms.

The term "sclerosis tuberosa" as used herein refers to a rare multisystem autosomal dominant genetic disease that causes non-cancerous tumors to grow in the brain and on other vital organs such as the kidneys, heart, liver, eyes, lungs and skin. A combination of symptoms may include seizures, intellectual disability, developmental delay, behavioral problems, skin abnormalities, lung disease, and kidney disease.

The term "MECP2 duplication syndrome" in the context of the present description refers to a rare disease that is characterized by severe intellectual disability and impaired motor function. It is an X-linked genetic disorder caused by the overexpression of MeCP2 protein. Symptoms of M2DS include infantile hypotonia and failure to thrive, delayed psychomotor development, impaired speech, abnormal or absent gait, epilepsy, spasticity, gastrointestinal motility problems, recurrent infections, and genitourinary abnormalities.

The expression "encephalic epilepsies post septic meningitis" as used herein refers to the rare complication of sepsis due to infection with bacterial meningitis, which leads to the appearance of epileptic episodes which are normally drug resistance.

The term "Dravet syndrome" as used herein refers to a severe form of epilepsy characterized by frequent, prolonged seizures often triggered by high body temperature (hyperthermia), developmental delay, speech impairment, ataxia, hypotonia, sleep disturbances, and other health problems.

The term "Lennox-Gastaut syndrome" as used herein refers to a complex, rare, and severe childhood-onset epilepsy. It is characterized by multiple and concurrent seizure types, cognitive dysfunction, and slow spike waves on electroencephalogram (EEG). Typically, it presents in children aged 3-5 years and can persist into adulthood.

The term "Doose syndrome", also known as Myoclonic atonic epilepsy (MAE), refers to a form of epilepsy that affects boys more than girls and starts in early childhood with the first seizure usually occurring between 2 to 6 years of age.

Development prior to seizure onset is usually normal. With frequent seizures, development slows and may regress.

The term "neuropathic pain" as used herein refers to the pain caused by damage or dysfunction of the central nervous system or peripheral nerve and non-inflammatory pain such as neuropathy due to drug therapy or radiotherapy. In the present invention the neuropathic pain is derived from leukodystrophy.

The term "spasticity" as used herein refers to a motor disorder characterized by velocity dependent increase in the tonic stretch reflex with exaggerated tendon jerks. In the present invention the spasticity is derived from leukodystrophy.

The term "leukodystrophy" as used herein refers to a group of disorders that are characterized by an abnormal formation, turnover, or destruction of myelin. In a particular embodiment of the treatment of the invention, the leukodystrophy is selected from a group consisting of: metachromatic leukodystrophy, Krabbe disease, X-Linked adrenoleukodystrophy, Canavan disease and Alexander disease. The term "metachromatic leukodystrophy" or "MLD", as used herein refers to a leukodystrophy which results from the genetic defects in the enzymes associated with the cellular compartment the lysosome. MLD is inherited in an autosomal recessive way and is the result of mutations in three different ARSA alleles that encode the enzyme arylsulfatase A (ASA or sometimes ARSA), also called sulfatide sulfatase.

The term "Krabbe disease" in the present context refers to another type of leukodystrophy with autosomal recessive inheritance that is the result of a lysosomal storage disorder. It is due to a deletion in exon 16 of the GALC gene that causes a frameshift mutation leading to a premature stop codon. The GALC gene, found on chromosome 14 at position 31 (14q31), codes for the enzyme beta-galactocerebrosidase (GALC).

The term "Canavan disease" as used herein refers to a type of leukodystrophy that, like MLD and Krabbe disease, is also passed on in an autosomal recessive inheritance pattern. It is due to a mutation in the ASPA gene that encodes aspartoacylase, an enzyme needed to metabolize N-acetyl-L-aspartate (NAA). The mutation causes a deficiency of aspartoacylase.

The term "X-linked adrenoleukodystrophy" or its acronym "X-ALD", refers to a pathology which results from a mutation in the peroxisomal ATP-binding cassette (ABC transporter). This leads to cerebral inflammatory demyelination caused by the myelin destabilization that occurs in these patients. In X-ALD patients, abnormally high levels of very long chain fatty acid (VLCFA) accumulate in various body tissues and fluids. This increased concentration then incorporates into various complex lipids where they are not normally found. This has been found to be directly involved in the cerebral inflammation.

The term "Alexander disease" as used herein refers to a unique from the leukodystrophies mentioned above in that it is the result of spontaneous mutation that is not inherited. Alexander disease results from the accumulation of Glial fibrillary acidic protein (GFAP) as the result of a mutation in the GFAP gene; which, rather than being found in association with lysosomes or peroxisomes, is an intermediate filament linked to the nuclear envelope.

The extract of the invention or the pharmaceutical composition of the invention can be formulated as best required to achieve the most effective therapeutic effect. In a particular embodiment of the medical use of the invention, the extract of the invention or the pharmaceutical composition of the invention is formulated for oral, topical or respiratory administration, preferably oral administration.

In another particular embodiment of the medical use of the invention, the extract of the invention or the pharmaceutical composition of the invention is administrated at least once a day, at least 2 times a day, at least 3 times a day, at least 4 times a day, at least 5 times a day, at least 6 times a day, at least 7 times a day, at least 8 times a day, at least 9 times a day, at least 10 times a day, at least 11 times a day, at least 12 times a day.

In another particular embodiment of the medical use of the invention, the extract of the invention or the pharmaceutical composition of the invention is administrated at least once a week, at least 2 times a week, at least 3 times a week, at least 4 times a week, at least 5 times a week, at least 6 times a week, at least 7 times a week.

In another particular embodiment of the medical use of the invention, the extract of the invention or the pharmaceutical composition of the invention is administrated at least once a week, at least 2 times a month, at least 3 times a month, at least 4 times a month, at least 5 times a month, at least 6 times a month, at least 7 times a month, at least 8 times a month, at least 9 times a month, at least 10 times a month, at least 11 times a month, at least 12 times a month, at least 13 times a month, at least 14 times a month, at least 15 times a month, at least 16 times a month, at least 17 times a month, at least 18 times a month, at least 19 times a month, at least 20 times a month, at least 21 times a month, at least 22 times a month, at least 23 times a month, at least 24 times a month, at least 25 times a month, at least 26 times a month, at least 27 times a month, at least 28 times a month, at least 29 times a month, at least 30 times a month, at least 31 times a month.

In another particular embodiment of the medical use of the invention, the extract of the invention or the pharmaceutical composition of the invention is administrated at least once a year, at least 2 times a year, at least 3 times a year, at least 4 times a year, at least 5 times a year, at least 6 times a year, at least 7 times a year, at least 8 times a year, at least 9 times a year, at least 10 times a year, at least 11 times a year, at least 12 times a year.

The invention will be described by way of the following examples.

EXAMPLES

**Example 1: Manufacturing Process and Process Controls**

OI4C is an herbal preparation obtained by extraction. Its manufacturing process consists of two stages. The first stage relates with the manufacture of Technical OI4C batches. The second stage relates with the combination of several Technical OI4C batches and the manufacturing of a single batch of OI4C (bulk).

The starting material is *Cannabis sativa* L. flowers, which are obtained from a *Cannabis sativa L.* plant of the variety ycj-01.

*Technical OI4C*

Cannabis flowers are dried and mixed with ice in extraction bags which are suspended in chilly water. After maceration under low temperature, the aqueous phase is eventually discarded by suction and filtration, obtaining the trichomes. The herbal cake is partially air-dried and stored in aluminum bags under argon at low pressure. These manufacturing operations can be repeated as many times as needed. A combination of several Technical OI4C batches can be used for the next stage.

*OI4C (bulk)*

Up to four batches of Technical OI4C are combined and mixed with ethanol under argon. The mixture is stirred at 30 °C, then at reflux for decarboxylation, which is checked by HPLC. The extract is then filtered and concentrated to dryness under vacuum, with control of the amount of residual ethanol. Then it is diluted with medium-chain triglycerides and filtered again. The extract is standardised for CBD. Finally, it is packaged in amber glass bottles under argon, and it is stored at 5 °C.

The following process narrative is for a typical batch of Technical OI4C starting from 6.0 kg of cannabis flowers.

1. Place cannabis flowers (FLCEO) (about 60 g) and ice (about 120 g) in extraction bags and store in a freezer.
2. Add potable water (60 L) to the reactor and stir cooling at 0-5 °C.
3. Load 50 bags of step 1 and ice cubes (60 kg) in the reactor.
4. Stir the mixture at 0-5 °C for 3 h. Then, collect and squeeze the bags.
5. Load 50 new bags of step 1 in the reactor.
6. Stir the mixture at 0-5 °C for 3 h. Then, collect and squeeze the bags.
7. Load 50 new bags of step 1 in the reactor.
8. Stir the mixture at 0-5 °C for 3 h. Then, collect and squeeze the bags.
9. Warm up the reactor mixture to room temperature. Then, stop stirring.
10. Non-critical in-process control CP-1
Check visually the mixture for the presence of ice.

- If the mixture does not contain ice, proceed to the next step.
- If the mixture contains ice, wait until the whole mixture is fully dissolved.

11. Collect and discard the supernatant aqueous solution.
12. Filter the content of the reactor in a Nutsche through a 23 $\mu$m filter and wash with potable water (2-3 L). Drain with argon pressure and under vacuum for 10-20 min. Eventually, discard the washings.
13. In a clean room, sieve the wet cake, spread on trays and air dry.
14. In-process control CP-2.
Check the mixture for loss on drying.

- If the mixture loss on drying is less than 10%, proceed to next step.
- If the mixture loss on drying is more than 10%, carry on drying until a loss on drying of less than 10% is achieved.

15. Pack the product in aluminum bags at low pressure and under argon. If necessary, store at $\leq$ 25 °C.
The current batch size of Technical OI4C is up to 1.1 kg.
16. Load ethanol (12 L) and four batches of Technical OI4C in the reactor under argon.
17. Stir at 30 °C for 48 h, then at 90-100 °C for 40-48 h.
18. In-process control CP-3.
Check the mixture for conversion.

**EP 4 306 121 B1**

- If CBD/(CBD+CBDA) is ≥ 98.0%, proceed to the next step.
- If CBD/(CBD+CBDA) is < 98.0%, keep on heating for ≥ 2 h and repeat CP-3.

19. Filter the suspension through a 50 μm filter in a Büchner-type filter and wash with ethanol (0.5 L).
20. Cool the filtrate at room temperature, then at ≤ 5 °C for ≥ 8 h.
21. Clarify the filtrate through a 5 μm filter in a Büchner-type filter and wash with ethanol (0.5 L).
22. Evaporate the solution in the rotary evaporator at 85 °C.
23. Add 200 g of MCT to the concentrate and keep on evaporating for ≥ 2 h.
24. In-process control CP-4.
Check the concentrate for residual solvents and assay (CBD).

- If the amount of ethanol is ≤ 1.0%, proceed to the next step.
- If the amount of ethanol is > 1.0%, keep on drying under the same conditions for ≥ 2 h and repeat CP-4.
- The CBD assay value (A) is for calculation purposes in the next steps. There is no acceptance criterion.

25. Weigh the extract (B).
26. Add MCT to the extract and stir the mixture for 5-10 min. The amount of MCT to be added is calculated from A and B.

$$Amount\ of\ MCT\ (C, grams) = \frac{B \times (A - 20)}{20}$$

27. Filter the extract under pressure and vacuum through a 50 μm filter, then through a 19 μm filter in a Nutsche filter. Wash with MCT. The amount of MCT to be added is calculated from A, B and C.

$$Amount\ of\ MCT\ (D, grams) = \frac{B \times (A - 17)}{17} - C$$

28. Filter the extract under pressure and vacuum through a 5 μm filter in a Nutsche filter. Wash with MCT. The amount of MCT to be added is calculated from A, B, C and D.

$$Amount\ of\ MCT\ (grams) = \frac{B \times (A - 14)}{14} - C - D$$

29. Stir the mixture for 5-10 min at room temperature.
30. In-process control CP-5.
Check the extract for assay (CBD, E).

- If the assay (CBD) is > 10.35% w/v, proceed to the next step.
- If the assay (CBD) is in the range 10.00-10.35% w/v, proceed to step 33.
- If the assay (CBD) is < 10.00% w/v, apply the non-conformance procedure.

31. Weigh the extract (F, grams).
32. Add MCT and stir for 5-10 min. The amount of MCT to be added is calculated from E and F.

$$Amount\ of\ MCT\ (g) = \frac{E \times (F - 10.35)}{10.35}$$

33. Pack the product in 1 L amber glass bottles. If dosing is not expected immediately afterwards, store at ≤ 5 °C under argon.

The current batch size of the herbal preparation is 2.0-2.6 kg.

Drug extract ratio (DER): 8.3-11.3:1 (based on 2 executed batches)
Genuine drug extract ratio (DER genuine): 38.6-53.5:1 (based on 2 executed batches)

14

*Reprocess*

The only reprocess considered for this manufacturing process is if OI4C fails to meet the assay specification when analyzed for CBD. In this case, and provided that the assay value is > 10.35%, it can be subjected to further dilution with MCT to meet the acceptance criterion. However, in-process control CP-5 is in place at the end of the manufacturing process to prevent this potential situation.

Reworking is not considered in any case.

*Assay (CBD), equivalent THC and CBN*

In-house procedure (Table 2).

Volumetric material is of class A.

All solvents and reagents for mobile phase preparation are of HPLC quality.

All solutions are filtered through a 0.45 $\mu$m nylon membrane filter prior to injection.

Table 1. In-house HPLC procedure for testing of flowers.

| Equipment | Agilent 1100 Series or equivalent | | |
|---|---|---|---|
| Flow | 0.4 mL/min | | |
| Temperature | 25 °C | | |
| Injection Volume | 5 $\mu$L | | |
| Wavelength | 228 nm (bandwidth: 4 nm) | | |
| Column | Agilent InfinityLab Poroshell 120 EC-C18 3.0 $\times$ 100 mm, 2.7 $\mu$m or equivalent | | |
| Mode | Gradient: Mobile phase A*/ Mobile phase B** | | |
| | Time (min) | % A | % B |
| | 0.00 | 30 | 70 |
| | 15.00 | 20 | 80 |
| | 15.01 | 5 | 95 |
| | 16.00 | 5 | 95 |
| | 16.10 | 30 | 70 |
| | 20.00 | 30 | 70 |
| Relative retention times | CBD: 1.00 CBDA: 0.83 CBG: 0.95 CBN: 1.56 $\Delta$9-THC: 1.93 $\Delta$8-THC: 1.98 $\Delta$9-THCA: 2.47 | | |
| * Mobile phase A: (Milli-Q water/ 0.1% formic acid): transfer 1 mL of formic acid to a graduated cylinder and make up to 1 L with Milli-Q water. ** Mobile phase B: (MeCN/ 0.1% formic acid): transfer 1 mL of formic acid to a graduated cylinder and make up to 1 L with acetonitrile. | | | |

Solutions

Prepare the following solutions. All solutions need to be filtered through a 0.45 $\mu$m nylon membrane filter prior to injection.

Blank: Use ethanol 96%.

Standard solution: Accurately weigh 50 mg of CBD WS in a 50 mL volumetric flask. Dissolve and make up to the mark with ethanol 96%. Transfer 10 mL to a 50 mL volumetric flask and make up to the mark with ethanol 96%.

Control solution: Prepare as described for Standard solution.

System suitability solution: Weigh 20 mg of THC WS in a 100 mL volumetric flask. Dissolve and make up to the mark with ethanol 96%. Transfer 5 mL to a 50 mL volumetric flask and make up to the mark with ethanol 96%.

Sample solution: Grind 10-50 g of the substance to be examined with a suitable mill and sieve the powder through a 710 pore sieve - the sample of the Identification (TLC) test is suitable for the preparation of this solution. In a 50 mL centrifuge tube shake 0.500 g of the powdered sample with 20 mL of ethanol 96% for 15 min at 300 rpm. Then, get the tube centrifuged for 5 min at 3000 rpm. Transfer the supernatant liquid to a 50 mL volumetric flask. Treat the herbal residue twice with 12.5 mL of ethanol 96%. Transfer the ethanol phases to the 50 mL volumetric flask and make up to the mark with additional ethanol 96%. Finally, transfer 5 mL to a 50 mL volumetric flask and make up to the mark with ethanol 96%. Prepare in triplicate.

## Sequence

Inject the Blank in triplicate, the Standard solution in triplicate, the Control solution, the System suitability solution and the three Sample solutions.

## System suitability

The third Blank chromatogram does not contain any peak at the relative retention times of the cannabinoids to be analyzed.

The resolution between Δ8-THC and Δ9-THC in the System suitability solution is $\geq$ 1.5.

The coefficient of variation of the areas that correspond to CBD in the three replicates of the Standard solution is $\leq$ 2.0%.

The recovery of the *Control solution* is in the range 98.0-102.0%.

## Calculations

Calculate the content of equivalent THC, CBD and CBN using the following equations:

$$\% \, THC = \frac{A_{THC}^{smp} \times C_{CBD}^{std} \times P_{CBD}^{std}}{A_{CBD}^{std} \times \dfrac{w^{smp} \times (1 - LoD)}{500} \times 0.91} + \frac{A_{THCA}^{smp} \times C_{CBD}^{std} \times P_{CBD}^{std} \times 0.877}{A_{CBD}^{std} \times \dfrac{w^{smp} \times (1 - LoD)}{500} \times 1.67}$$

$$\% \, CBD = \frac{A_{CBD}^{smp} \times C_{CBD}^{std} \times P_{CBD}^{std}}{A_{CBD}^{std} \times \dfrac{w^{smp} \times (1 - LoD)}{500} \times 1.00} + \frac{A_{CBDA}^{smp} \times C_{CBD}^{std} \times P_{CBD}^{std} \times 0.877}{A_{CBD}^{std} \times \dfrac{w^{smp} \times (1 - LoD)}{500} \times 1.78}$$

$$\% \, CBN = \frac{A_{CBN}^{smp} \times C_{CBD}^{std} \times P_{CBD}^{std}}{A_{CBD}^{std} \times \dfrac{w^{smp} \times (1 - LoD)}{500} \times 2.34}$$

Where:

$A^{smp}{}_{THC}$ is the area that corresponds to Δ9-THC in the *Sample solution* chromatogram.

$A^{smp}{}_{THCA}$ is the area that corresponds to THCA in the *Sample solution* chromatogram.

$A^{smp}{}_{CBD}$ is the area that corresponds to CBD in the *Sample solution* chromatogram.

$A^{smp}{}_{CBDA}$ is the area that corresponds to CBDA in the *Sample solution* chromatogram.

$A^{smp}{}_{CBN}$ is the area that corresponds to CBN in the *Sample solution* chromatogram.

$C^{std}{}_{CBD}$ is the concentration of CBD WS in mg/mL in the *Standard solution.*

$P^{std}{}_{CBD}$ is the per cent purity factor of CBD WS.

$A^{std}{}_{CBD}$ is the average area that corresponds to CBD WS in the three *Standard solution* chromatograms.

$w^{smp}$ *is the mass of the sample.*

The assay (CBD) is calculated for the three samples and given as the average value.

**Example 2: CBD+THC intake (RMD): Observational patient study**

Composition per 5 ml: 4 g of olive oil, 1g of rmd oil.
The rmd oil per gram has 700 mg of cbd, 30 mg of thc and other flavonoids and cannabinoids. A summary of the results is shown on Figure 1.

1. Patient: V.L.M.

Date of birth and sex: 03/01/2013. Female

Disease: refractory epilepsy and psychomotor retardation.
Number of seizures BEFORE taking RMD: periods of 5, 30, 80 or more seizures per day depending on the medication. They are more or less intense head falls, not in bursts. When sleeping he also has some myoclonia.
Antiepileptics BEFORE taking RMD: Depakine. Zonisamide. Keppra, vimpat, rivotril, zarontin, noiafren, phenobarbital, ketogenic diet, lamictal, ethosuximide (zarontin).
CBD USE:

Time using CBD: 20 months not continuous
Has taken CBD from different sources, hemp, industrial etc. with no relevant positive effect on seizures.
Time using RMD: 7 Months and ongoing
After almost three months of use, he began to notice an improvement, although he also had a change in the type of seizures, having tonic seizures with respiratory involvement during sleep of no more than 20 seconds once a week for a month. Then the frequency decreased to every 10 days, then every 15 days, and now for 2 months he has been free of these seizures. He also had myoclonus when he fell asleep, of which he still has some sporadic myoclonus.
Since the middle of March, the head falls that were his characteristic crises have disappeared. His doses are still being adjusted.
Antiepileptics DURING RMD use: ZARONTIN and DEPAKINE at present, but in lower doses than recommended by his doctors.

2. Patient: H.A.C

Date of birth and sex: 16/03/2013. Male
Disease: Chromosomopathy, history of West syndrome, psychomotor retardation, mitochondrial deficit.
Number of seizures BEFORE taking RMD: 5 Seizures per day.
Antiepileptics BEFORE taking RMD: ACTH intramuscular Depakine. Keppra, vigabatrin, intravenous immunoglubin, ketogenic diet.
CBD USE:
Time using CBD: for 11 months he has taken industrial CBD from different sources without significant changes.

For the last seven months, he has been taking RMD.
Currently the patient has a daily seizure.
He does not take any other anti-epileptic medication.

3. Patient: M.M.P

Date of birth and sex: 17/08/2006. Female
Disease: Tuberous Sclerosis.
Number of seizures BEFORE taking RMD: 5 -10 daily seizures.
Antiepileptics BEFORE taking RMD: Inovelon, noiafren, sabrilex, depakine everolimus.
USE OF CBD:
Time using CBD: 3 months using CBD plus which reduced seizures to 2-3 per day.

For the last two months he has been taking RMD without being able to adjust his dose due to lack of supply.

Currently the patient has one seizure a week and is still being treated with Inovelon, Noiafren, Sabrilex.

4. Patient: P.B.M

Date of birth and sex: 30/05/2009. Male
Disease: MECP2 duplication syndrome.
Number of seizures BEFORE taking RMD: 3-5 daily seizures.
Antiepileptic drugs BEFORE taking RMD: Depakine, Keppra, sabrilex.
Time using RMD: 5 Months. The patient has never taken CBD exclusively. He has been on RMD since the beginning of treatment.

The seizures have been reduced to 1-2 alternate days, despite not being able to administer the right dose for him due to a shortage of product.

He is also still being treated with Depakine and Kepra.

5. Patient: M.B.M

Date of birth and sex: 21/11/2006. Male
Disease: MECP2 duplication syndrome.
Number of seizures BEFORE taking RMD: 4-5 daily seizures.
Antiepileptics BEFORE taking RMD: Depakine, Lamictal, Rivotril, Zonegran Kepra, sabrilex, intramuscular ACTH.
CBD USE:

9 Months. No significant change in the patient.
Time using RMD: 8 Months. Despite taking a low dose, due to product shortage, seizures have been reduced to 3 per day with seizure-free periods of up to five consecutive days.
Antiepileptics during RMD: Lamictal, Vimpat, Rivotril.

6. Patient: A.J.G

Date of birth and sex: 17/06/2008. Male
Illness: Severe epilepsy. West history.
Number of seizures BEFORE taking RMD: 25-27 daily seizures between 15 spasms, tonic and atonic.
Antiepileptics BEFORE taking RMD: Zonegran Kepra, Tegretol, Sinogan.
CBD USE:

3 Months. No significant change in the patient.
Time using RMD: 11 Months. Presents 3 atonic tonic crises, every other day. Antiepileptics during RMD: Tegretol, Keppra, Zonegan.

Claims

1. A method for obtaining a cannabis extract comprising:

a) obtaining trichomes from cannabis flowers wherein step a) comprises:

(i) collecting and drying the cannabis flowers from *Cannabis* plants;
(ii) cooling the cannabis flowers to a temperature of between 0°C to 10°C, and;
(iii) washing the cannabis flowers in water at a temperature between 0°C to 5°C as to separate the trichomes form the remaining flower;

b) drying the trichomes obtained in step a) to a moisture content of less than 10% by weight;

c) macerating the dried trichomes obtained in step b) with a C1 - C3 alcohol at a temperature of 30°C for at least 48 h, obtaining a first solution;

d) incubating and stirring the trichomes obtained in step c) with a C1-C3 alcohol at a temperature of 90°C to 100°C for at least 40 hours, obtaining a second solution;

e) eliminating at least part of the alcohol present in the second solution, obtaining an extract,

wherein the extract comprises at least 10% w/v of cannabidiol (CBD) and a concentration of less than 0.5% w/v of tetrahydrocannabinol, and wherein no purification is carried out on the extract.

2. The method according to claim 1 wherein the drying of step b) is performed at a temperature between 30°C to 35°C for 48 hours.

3. The method according to claim 1 or 2, wherein the cannabis flowers on step a) are from the species *Cannabis sativa L.*

4. The method according to any one of claims 1 to 3, wherein the cannabis flowers are from a cannabis plant of chemotype II or chemotype III.

5. The method according to any one of claims 1 to 4, wherein the extract comprises 1% v/v alcohol or less.

6. The method according to any one of claims 1 to 5, wherein the extract comprises a concentration of 10.00 % w/v of CBD to 10.35% w/v of CBD, preferably 10% w/v of CBD.

7. The method according to any one of claims 1 to 6, wherein if the extract has a concentration of more than 10.35 % w/v of CBD a further step of diluting the extract with an adequate excipient is carried out.

8. A cannabis extract derived from *Cannabis sativa* L. obtained by the method according to any one of claims 1 to 7.

9. The cannabis extract according to claim 8, wherein the extract comprises at least 10 % w/v of CBD and less than 0.5 % w/v of tetrahydrocannabinol.

10. A pharmaceutical composition comprising the cannabis extract according to claim 8 or 9 in a therapeutically effective dose and an adequate excipient and/or adjuvant.

11. The pharmaceutical composition according to claim 10, wherein the therapeutically effective dose is of 2.3 mg per kilo per day of CBD and 0.11 mg per kilo per day of tetrahydrocannabinol.

12. The cannabis extract according to claim 8 or 9 for use as a medicine.

13. The cannabis extract according to claim 8 or 9 or the pharmaceutical composition according to claim 12 or 13 for use in the prevention and/or treatment of refractory epilepsy and/or of neuropathic pain and spasticity derived from leukodystrophy.

**Patentansprüche**

1. Eine Methode zur Gewinnung eines Cannabisextrakts umfassend:

a) Gewinnung von Trichomen aus Cannabisblumen, wobei Schritt a) umfasst:

(i) Sammeln und Trocknen der Cannabisblüten von Cannabispflanzen;
(ii) kühlen der Cannabisblüten auf eine Temperatur zwischen 0°C und 10°C und;
(iii) waschen der Cannabisblüten in Wasser bei einer Temperatur zwischen 0°C und 5°C, um die Trichome von der übrigen Blüte zu trennen;

b) Trocknung der in Schritt a) erhaltenen Trichome auf einen Feuchtigkeitsgehalt von weniger als 10 % nach Gewicht;
c) Mazeration der getrockneten Trichome, die in Schritt b) gewonnen wurden, mit einem C1-C3-Alkohol bei einer Temperatur von 30°C für mindestens 48 Stunden, wobei eine erste Lösung erhalten wird;

d) Inkubation und Rühren der in Schritt c gewonnenen Trichome mit einem C1-C3-Alkohol bei einer Temperatur von 90°C bis 100°C für mindestens 40 Stunden, wobei eine zweite Lösung erhalten wird;

e) Entfernung mindestens eines Teils des in der zweiten Lösung vorhandenen Alkohols, wobei ein Extrakt erhalten wird,

wobei der Extrakt mindestens 10 % w/v Cannabidiol (CBD) und eine Konzentration von weniger als 0,5 % W/V Tetrahydrocannabinol umfasst und wobei keine Reinigung des Extrakts erfolgt.

2. Die Methode nach Anspruch 1, wobei die Trocknung von Schritt b) bei einer Temperatur zwischen 30°C und 35°C für 48 Stunden durchgeführt wird.

3. Die Methode nach Anspruch 1 oder 2, wobei die Cannabisblüten in Schritt a) von der Art *Cannabis sativa L* stammen.

4. Die Methode nach einem der Ansprüche 1 bis 3, wobei die Cannabisblüten von einer Cannabispflanze des Chemotyps II oder Chemotyps III stammen.

5. Die Methode nach einem der Ansprüche 1 bis 4, wobei der Extrakt 1 % v/v Alkohol oder weniger umfasst.

6. Die Methode nach einem der Ansprüche 1 bis 5, wobei der Extrakt eine Konzentration von 10,00 % w/v CBD bis 10,35 % w/v CBD, vorzugsweise 10 % w/v CBD, umfasst.

7. Die Methode nach einem der Ansprüche 1 bis 6, wobei bei einer Konzentration von mehr als 10,35 % w/v CBD ein weiterer Schritt zur Verdünnung des Extrakts mit einem geeigneten Hilfsstoff durchgeführt wird.

8. Ein Cannabisextrakt, der aus *Cannabis sativa* L. gewonnen wurde, gemäß einem der Ansprüche 1 bis 7.

9. Der Cannabisextrakt nach Anspruch 8, wobei der Extrakt mindestens 10 % w/v CBD und weniger als 0,5 % w/v Tetrahydrocannabinol enthält.

10. Eine pharmazeutische Zusammensetzung, umfassend den Cannabisextrakt gemäß Angabe 8 oder 9 in einer therapeutisch wirksamen Dosierung und einem geeigneten Hilfsstoff und/oder Adjuvans.

11. Die pharmazeutische Zusammensetzung nach Anspruch 10, wobei die therapeutisch wirksame Dosis 2,3 mg pro Kilogramm pro Tag CBD und 0,11 mg pro Kilo pro Tag Tetrahydrocannabinol beträgt.

12. Der Cannabisextrakt nach Anspruch 8 oder 9 zur Verwendung als Medikament.

13. Der Cannabisextrakt nach Anspruch 8 oder 9 oder die pharmazeutische Zusammensetzung nach Anspruch 12 oder 13 zur Verwendung in der Prävention und/oder Behandlung von refraktärer Epilepsie und/oder neuropathischen Schmerzen und Spastizität infolge von Leukodystrophie.

## Revendications

1. Procédé pour obtenir un extrait de cannabis, comprenant :

a) obtenir des trichomes à partir de fleurs de cannabis, l'étape a) comprenant :

(i) collecter et sécher les fleurs de cannabis provenant de plantes de *Cannabis,*
(ii) refroidir les fleurs de cannabis à une température comprise entre 0 °C et 10°C,
(iii) laver les fleurs de cannabis dans de l'eau à une température comprise entre 0 °C et 5 °C de manière à séparer les trichomes de la fleur restante,

b) sécher les trichomes obtenus à l'étape a) jusqu'à une teneur en humidité inférieure à 10 % en poids,
c) faire macérer les trichomes séchés obtenus à l'étape b) avec un alcool C1 - C3 à une température de 30 °C pendant au moins 48 h, en obtenant une première solution,
d) incuber et agiter les trichomes obtenus à l'étape c) avec un alcool C1 - C3 à une température de 90 °C à 100 °C pendant au moins 40 heures, en obtenant une seconde solution,

e) éliminer au moins une partie de l'alcool présent dans la seconde solution, en obtenant un extrait,

dans lequel l'extrait comporte au moins 10 % p/v de cannabidiol (CBD) et une concentration inférieure à 0,5 % p/v de tétrahydrocannabinol, et dans lequel aucune purification n'est effectuée sur l'extrait.

2. Procédé selon la revendication 1, dans lequel le séchage de l'étape b) est effectué à une température comprise entre 30 °C et 35 °C pendant 48 heures.

3. Procédé selon la revendication 1 ou 2, dans lequel les fleurs de cannabis de l'étape a) proviennent de l'espèce *Cannabis sativa L.*

4. Procédé selon l'une quelconque des revendications 1 à 3, dans lequel les fleurs de cannabis proviennent d'une plante de cannabis de chimiotype II ou de chimiotype III**.**

5. Procédé selon l'une quelconque des revendications 1 à 4, dans lequel l'extrait comporte 1 % v/v d'alcool ou moins.

6. Procédé selon l'une quelconque des revendications 1 à 5, dans lequel l'extrait comporte une concentration de 10,00 % p/v de CBD à 10,35 % p/v de CBD, de préférence 10 % p/v de CBD.

7. Procédé selon l'une quelconque des revendications 1 à 6, dans lequel, si l'extrait a une concentration supérieure à 10,35 % p/v de CBD, une étape supplémentaire de dilution de l'extrait avec un excipient approprié est réalisée.

8. Extrait de cannabis dérivé de *Cannabis sativa L.* obtenu par le procédé selon l'une quelconque des revendications 1 à 7.

9. Extrait de cannabis selon la revendication 8, dans lequel l'extrait comporte au moins 10 % p/v de CBD et moins de 0,5 % p/v de tétrahydrocannabinol.

10. Composition pharmaceutique comprenant l'extrait de cannabis selon la revendication 8 ou 9 dans une dose thérapeutiquement efficace et un excipient et/ou adjuvant approprié.

11. Composition pharmaceutique selon la revendication 10, dans laquelle la dose thérapeutiquement efficace est de 2,3 mg par kilo par jour de CBD et de 0,11 mg par kilo par jour de tétrahydrocannabinol.

12. Extrait de cannabis selon la revendication 8 ou 9, destiné à un usage médical.

13. Extrait de cannabis selon la revendication 8 ou 9 ou composition pharmaceutique selon la revendication 12 ou 13 pour une utilisation dans la prévention et/ou le traitement d'une épilepsie réfractaire et/ou d'une douleur neuropathique et d'une spasticité découlant d'une leucodystrophie.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021238117 A1

**Non-patent literature cited in the description**

- *International Convention for the Protection of New Varieties of Plants (UPOV treaty),* 02 December 1961

- **PETILKA et al.** *Helv. Chim. Acta*, 1969, vol. 52, 1102
- **MECHOULAM et al.** *J. Am. Chem. Soc*, 1965, vol. 87, 3273